# EUROPEAN PATENT APPLICATION

(11) **EP 3 671 208 A1**
(43) Date of publication of application: **24.06.2020**
(21) Application number: 18215047.4
(22) Date of filing: 21.12.2018
(51) Int. Cl.: G01N 33/53, G01N 23/2251

(54) **IGE DRIVEN STRUCTURE DETERMINATION OF TARGET MOLECULES USING CRYO-EM**

(71) Applicant: Aarhus Universitet, 8000 Aarhus C (DK)
(72) Inventor: Andersen, Gregers, Rom, 8220 Brabrand (DK); Spillner, Edzard, 8270 Højbjerg (DK)
(74) Representative: Plougmann Vingtoft a/s

(57) **Abstract**

The present invention relates to a method for determining the structure of a target molecule, the method comprising binding an IgE antibody or fragment derivative thereof to the target molecule, thereby producing a complex of the IgE antibody or fragment or derivative thereof and the target molecule; and determining the structure of the target molecule using preferably cryo-electron microscopy. The invention also relates to agents which can induce global two-fold symmetry of a complex between the IgE and the target molecule.

## Description

### Technical field of the invention

The present invention relates to improved structure determination of molecules using EM, preferably cryo-EM, where IgE or fragments or derivatives thereof are used to increase rigidity and/or induce local two-fold symmetry and/or induce global symmetry. In particular, the present invention relates to structure determination of small proteins using cryo-EM.

### Background of the invention

Proteins are essential to living organisms where they function e.g. as enzymes, transporters, structural components and transmitters of signals. Crystallography is the major tool for structure determination but requires crystallization of the protein. This is demanding in terms of time and cost and often impossible for flexible, non-globular and unstable proteins and large protein complexes. Recent developments have enabled structure determination of proteins by cryo electron microscopy (cryo-EM) comparable in resolution (1.5-5 Å) to those attainable by crystallography. However, determination by cryo-EM is currently not trivial. Preparation of cryo-EM grids with individually resolved protein molecules is not straight forward and access to microscopes is limited. A general approach facilitating and accelerating cryo-EM structure determination of proteins is therefore urgently required. In contrast, very fast EM reconstructions may be achieved with negative stain EM (nsEM), but here the resolution is limited. Cryo-EM works best with proteins having a molecular weight >200 kDa, since it relies on averaging thousands of low-contrast images of single molecules, and the signal-to-noise ratio in the data will increase with protein size and compactness. Symmetry within the investigated protein is also beneficial since internal averaging can then be applied.

Hence, an improved method for structure determination using cryo-EM would be advantageous, and in particular a more efficient and/or reliable method for structure determination of small proteins would be advantageous.

### Summary of the invention

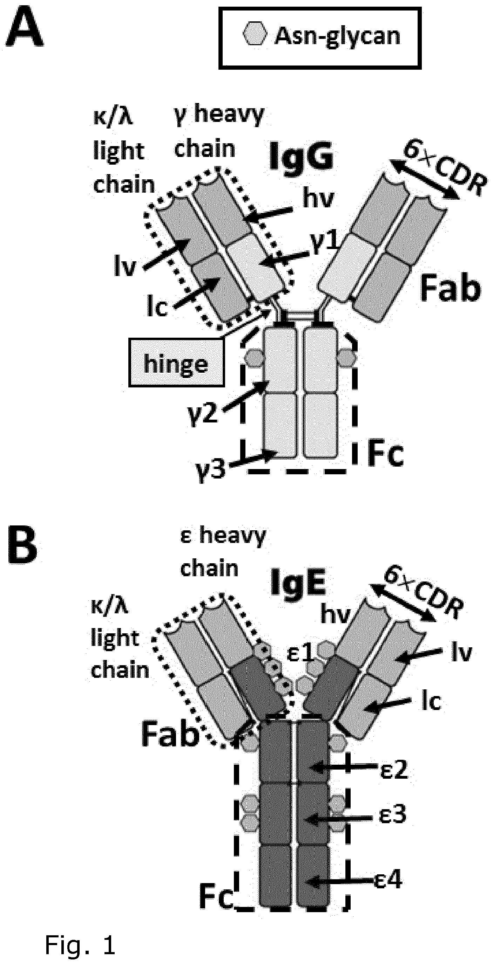

Antibody recognition of antigens is central to immunity. There are five different antibody classes: IgG, IgM, IgE, IgD, IgA. Their basic architecture is the same: Two identical fragments called Fab connect to an Fc fragment. The antigen (Ag) is recognized by six complementarity determining regions (CDRs) on each Fab. IgG, IgA, IgM, IgD are flexible at the Fab-Fc junction (the hinge region, Fig 1A). This flexibility facilitates bridging of two antigens and probably explains why there are only five available crystal structures and six EM structures of intact antibodies. The flexibility has also been confirmed by X-ray solution scattering for IgG, IgA and IgM. IgG-antigen complexes are therefore flexible, not compact and lack global two-fold symmetry. In contrast, an nsEM study of IgE revealed molecules that look quite compact compared to EM micrographs of IgG, IgA, IgM, IgD (Hui GK, et al. (2015) Biochem J 471(2): 167-185). The 170 kDa IgE contains an additional domain replacing the hinge compared to IgG (Fig 1A-B). In this larger IgE the Fc flexes between acutely bent and extended conformations (Drinkwater N, et al. Immunol Rev. 2015 Nov;268(1):222-35). Structural and biophyscial studies indicate that the IgE Fc fragments can adopt either a bent conformation with local two-fold symmetry where the ε3 domains are approaching and even may interact with the ε2 domains or an extended conformation with global two-fold symmetry where the ε2 and the ε3 domains are widely separated (Drinkwater N, et al., Immunol Rev. 2015 Nov;268(1):222-35).

Thus, an object of the present invention relates to the provision of a method for structural determination of target molecules using cryo-EM.

In particular, it is an object of the present invention to provide a method for structure determination of target molecules using cryo-EM that solves the above-mentioned problems of the prior art with determination of small target molecules.

The present invention relates to a method for determining the structure of a target molecule, the method comprising binding an IgE antibody or fragment derivative thereof to the target molecule, thereby producing a complex of the IgE antibody or fragment or derivative thereof and the target molecule; and determining the structure of the target molecule using preferably cryo-electron microscopy.

Thus, one aspect of the invention relates to a method for determining the structure of a target molecule and/or the structure of an IgE antibody interacting with the target molecule and/or the structure of the binding site between an IgE antibody and the target molecule, the method comprising
- providing a target molecule for which the structure is to be determined;
- providing an IgE antibody or fragment or derivative thereof, with binding affinity for the target molecule;
- binding the IgE antibody or fragment or derivative thereof to the target molecule, thereby producing a complex of the IgE antibody or fragment or derivative thereof and the target molecule; and
- determining the structure of at least the target molecule and/or the structure of an IgE antibody interacting with the target molecule and/or the structure of the binding site between the IgE antibody and the target molecule using electron microscopy, preferably cryo-electron microscopy.

As also discussed further below in the example section, example 2 shows that surprisingly IgE antibodies have a very rigid structure. Example 3 shows that such a rigid structure is also present when the IgE antibody is bound to an antigen (exemplified by GFP). Such rigid structures are highly advantageous when performing structure determination using EM. Example 6 shows how structure determination using the method of the invention are performed for cryo-EM.

In a preferred embodiment, said complex further comprises a binding agent for an ε-domain of IgE, such as for the ε2 and/or the ε3 domain and/or the ε4 domain, preferably for the ε2, and/or the ε3 domain. Example 4 shows that when the IgE antibody is bound to a molecule interacting with CHε3 (exemplified by the ligelizumab Fab), apparent global two-fold symmetry is obtained rather than the local two-fold symmetry observed for IgE and IgE-GFP. Example 5 shows global two-fold symmetry of a complex of IgE-GFP-ligelizumab.

Another aspect of the present invention relates to the use of an IgE antibody or derivative or fragment thereof as a binding agent to a target molecule for which the structure is to be determined by electron microscopy.

Yet another aspect of the present invention relates to the use of a binding agent for an ε-domain of IgE, such as for the ε2 and/or the ε3 domain and/or the ε4 domain, preferably for the ε2, and/or the ε3 domain, for inducing global two-fold symmetry of a complex of the IgE and target molecule bound thereto.

Still another aspect of the present invention is to provide a cryo-EM grid loaded with a complex of IgE antibody or fragment or derivative thereof and a target molecule.

### Brief description of the figures

Figure 1 shows the principles of antibody architecture. A-B) Comparison of IgG and IgE with domains and fragments named.

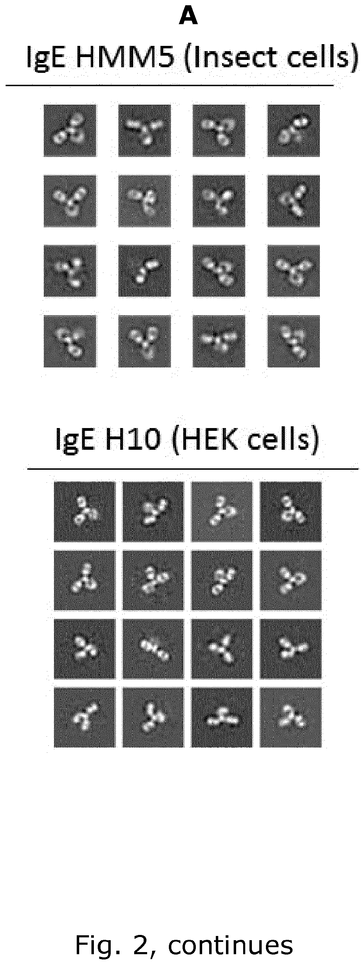

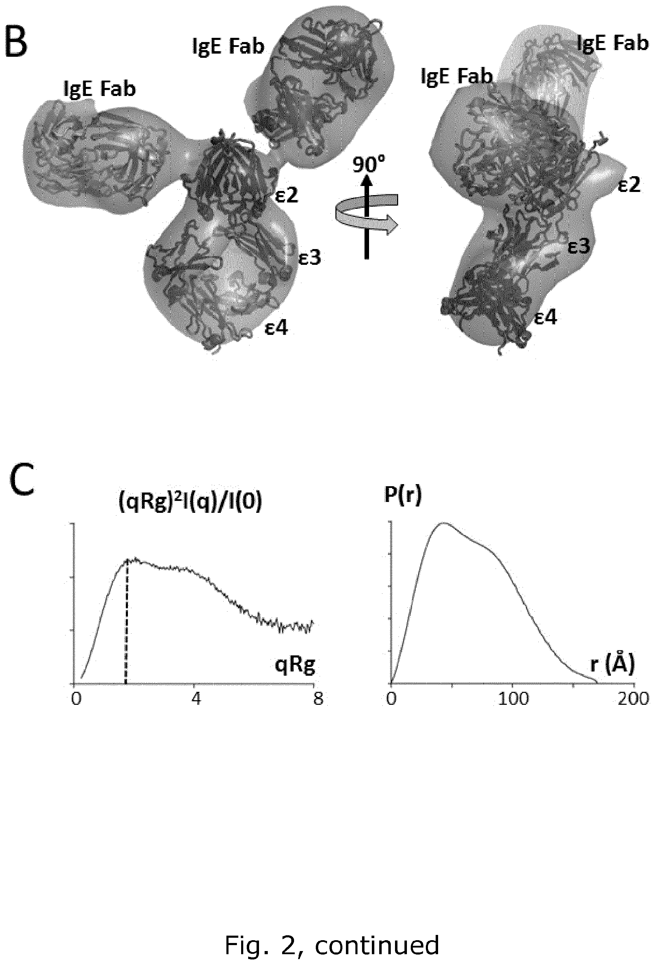

Figure 2 presents structure analysis of intact IgE. **A)** Negative stain EM 2D classes obtained from two different IgE molecules (HMM5 and H10) suggest that IgE rigidity is a common property. **B)** The 3D reconstruction for the HMM5 IgE with an IgE model fitted. Notice that the volume containing the Fab is comparable to the volume for the ε3-ε4 domain, in agreement with that both contain 4 Ig domains and that the Fabs are rigidly attached to their ε2 domain. At this resolution (∼30 Å) the heavy and light chain of the Fabs cannot be distinguished. **C)** SAXS analysis of the HMM5 IgE. To the left is displayed a Guinier normalized Kratky plot of the data with a maximum at 1.7 (dotted line) confirming that it is a rigid particle in solution and not flexible. To the right the resulting pair distance distribution suggesting a maximum dimension of 170 Å for IgE in solution.

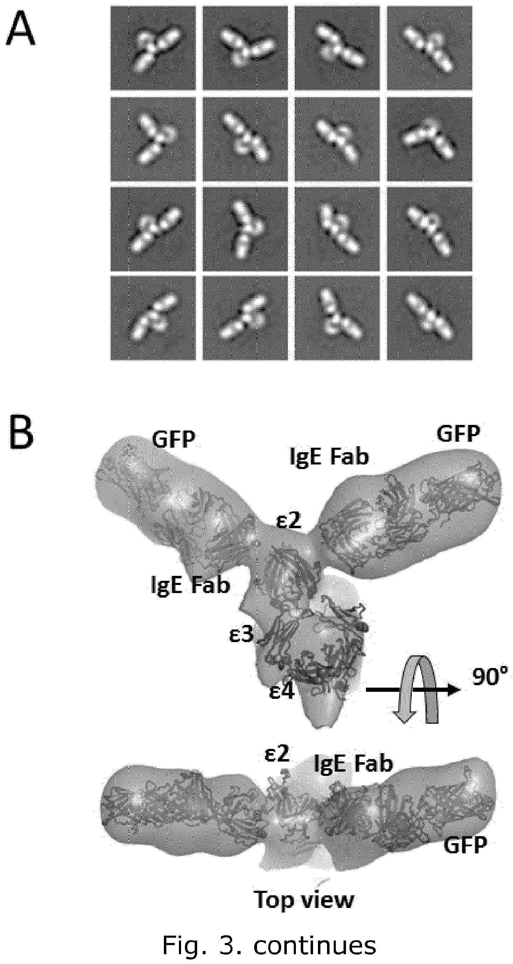

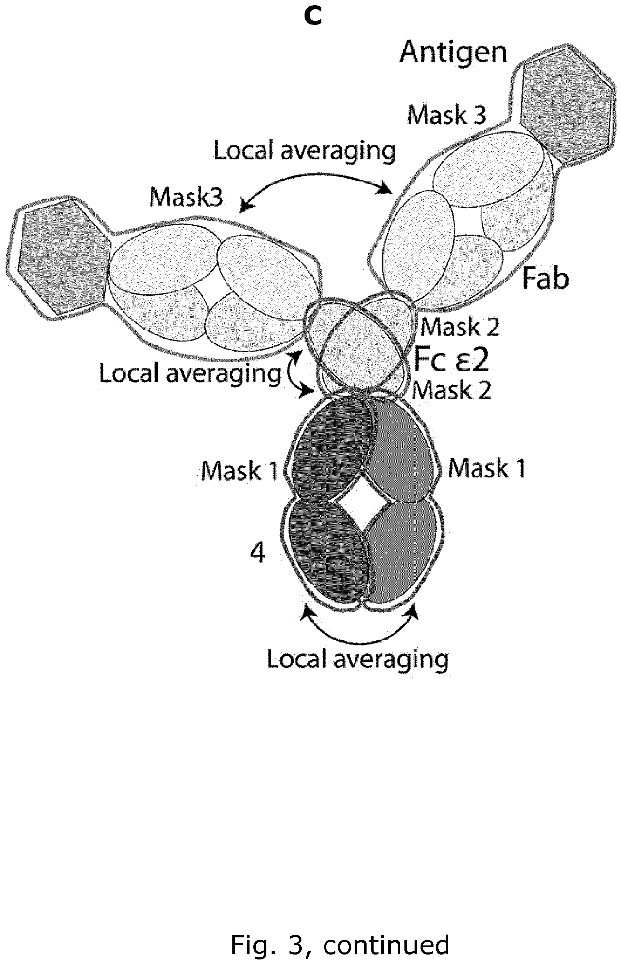

Figure 3 illustrates structure analysis of IgE bound to the model antigen GFP and the principle of local two-fold averaging. **A)** The nsEM 2D classes obtained from the IgE-GFP complex. Comparison with the 2D classes in Fig 2A directly shows additional volume at the distal end of each Fab for GFP. **B)** The 3D reconstruction of IgE-GFP. Notice that the volume around the GFP is compatible with a 30 kDa protein as compared to the 50 kDa contained within the Fab volume. The reconstruction is of lower quality for the Fc ε3-ε4 domains, possibly due to an underrepresentation of certain orientations of the IgE-GFP complexes on the grid. **C)** Smaller parts of IgE related by local two-fold symmetry can be masked separately and averaged to decrease the noise in the cryo-EM density maps and increase the reliability in particle alignment.

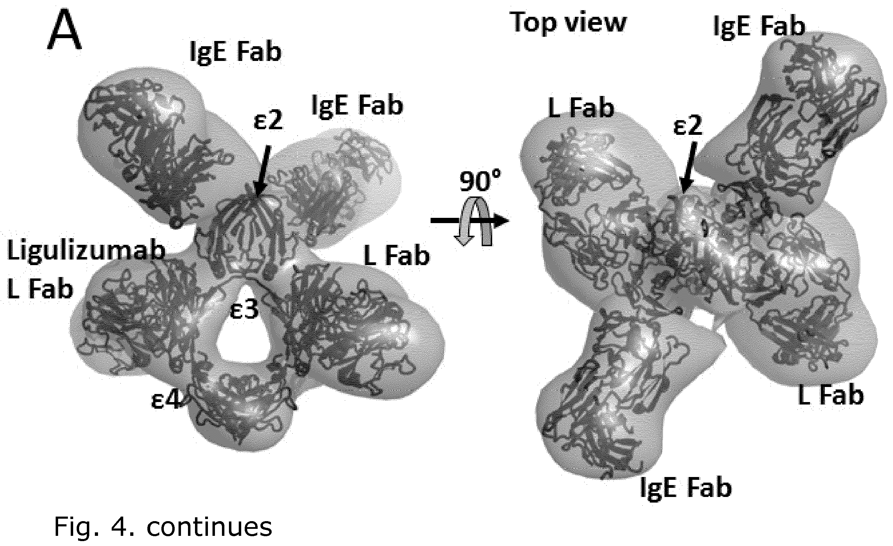

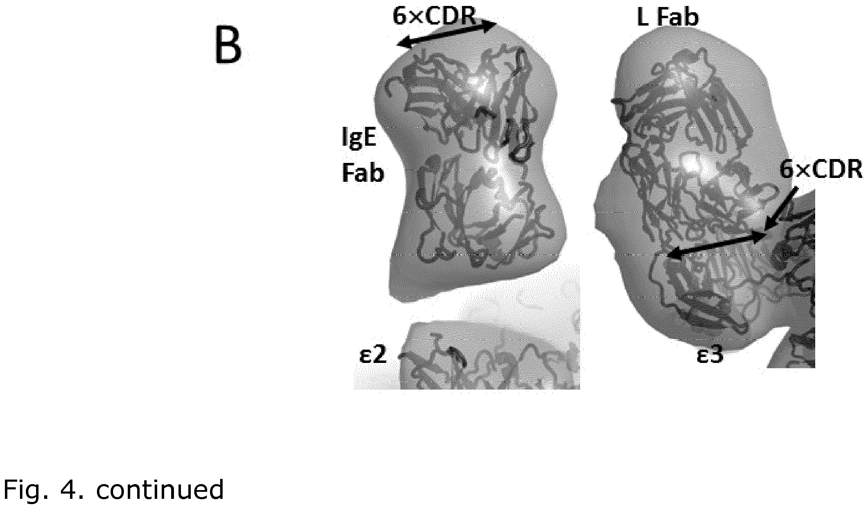

Figure 4 shows how binding of two ligelizumab Fab fragments induces an IgE conformation with apparent global two-fold symmetry. **A)** 3D reconstruction of the IgE-ligelizumab complex. **B)** Close-up comparing the two different Fab's present in the complex. To the left is the Fab of IgE itself, and to the right the ligelizumab Fab. Notice that their volumes are comparable suggesting that they are equally well ordered in the IgE-ligelizumab complex. In this case overall two-fold averaging was used during 3D reconstruction.

The present invention will now be described in more detail in the following.

### Detailed description of the invention

### Definitions

Prior to discussing the present invention in further details, the following terms and conventions will first be defined:

### Immunoglobulin E (IgE)

Immunoglobulin E (IgE) is a type of antibody (or immunoglobulin (Ig) "isotype") that has only been found in mammals. IgE is naturally synthesised by plasma cells. Monomers of IgE consist of two heavy chains (ε chain) and two light chains, with the ε chain containing 4 Ig constant domains (Cε1-Cε4 (figure 1B). This is in contrast to IgG which only contains 3 constant domains (Cγ1-Cγ3) (Figure 1A). Current models of intact IgE state that the Fc moiety comprising the three Ig domains ε2-ε4 is strongly bend with the ε3 domains approaching the ε2 domains and that one of the Fab's is located towards the bend Fc (Sutton BJ & Davies AM (2015). Immunol Rev 268(1):222-235).

IgE's main natural function is supposed to be immunity to parasites such as helminths like Schistosoma mansoni, Trichinella spiralis, and Fasciola hepatica. IgE is also utilized during immune defense against certain protozoan parasites such as Plasmodium falciparum.

IgE also has an essential role in type I hypersensitivity, which manifests in various allergic diseases, such as allergic asthma, most types of sinusitis, allergic rhinitis, and specific types of chronic urticaria and atopic dermatitis. IgE plays a pivotal role in responses to allergens, including but not limited to drugs, insect venoms, food, ect. and antigen preparations used in desensitization immunotherapy.

### Transmission electron microscopy (EM)

Transmission electron microscopy (EM) is a versatile technique that can be used to image biological specimens ranging from intact eukaryotic cells to individual proteins. There are several strategies for preparing samples for imaging by EM, including negative staining and cryogenic freezing.

### Crvo-Electron Microscopy (Crvo-EM)

Cryo-Electron Microscopy or cryo-EM is an electron microscopy (EM) technique applied on samples cooled to cryogenic temperatures and embedded in an environment of vitreous water. An aqueous sample solution is applied to a grid-mesh and quickly frozen to avoid ice formation.

### Negative stain EM

Negative stain EM is a method where an aqueous sample is applied to a grid-mesh and subsequently embedded in a heavy metal salt. The heavy metal salt is often derived from uranium, tungstate or molybdenum, and is used to increase the contrast of the specimen of interest.

### Equilibrium dissociation constant - K_{D}

K_{D} is the equilibrium dissociation constant, a ratio of k_{off}/kₒₙ, between the antibody and its antigen. K_{D} and affinity are inversely related. The K_{D} value relates to the concentration of antibody (the amount of antibody needed for a particular experiment) and so the lower the K_{D} value (lower concentration) and thus the higher the affinity of the antibody.

### Local two-fold symmetry

If a molecule contains two copies of a protomer molecule, the equivalent parts are related by local symmetry defined with symmetry operators. If these symmetry operators (as described by rotation and translation matrices) for the different parts are different, the symmetry is local. Local symmetry averaging can be used to improve the resolution of a reconstruction (the 3D density) obtained from cryo-EM micrographs.

### Global two-fold symmetry

If a molecule contains two copies of a protomer molecule and the equivalent parts are related by a single symmetry operator (as described by rotation and translation matrix) the symmetry is global. Global symmetry averaging can be used to improve the resolution of a reconstruction (the 3D density) obtained from cryo-EM micrographs and is technically simpler and more powerful than local symmetry averaging.

### Ligelizumab

Ligelizumab is a humanized monoclonal antibody designed for anti-IgE treatment of allergic diseases such as allergy, allergic asthma and chronic spontaneous urticaria.

### Method for determining the structure of a target molecule

As outlined above, the present invention is built on the rationale that surprisingly IgE antibodies adopt a very rigid structure and that this rigidity can be used to improve structure determination of target molecules in EM and in particular in cryo-EM. Thus, an aspect of the invention relates to a method for determining the structure of a target molecule and/or the structure of an IgE antibody interacting with the target molecule and/or the structure of the binding site between an IgE antibody and the target molecule, the method comprising
- providing a target molecule for which the structure is to be determined;
- providing an IgE antibody or fragment or derivative thereof, with binding affinity for the target molecule;
- binding the IgE antibody or fragment or derivative thereof to the target molecule, thereby producing a complex of the IgE antibody or fragment or derivative thereof and the target molecule; and
- determining the structure of at least the target molecule and/or the structure of an IgE antibody interacting with the target molecule and/or the structure of the binding site between the IgE antibody and the target molecule using electron microscopy, preferably cryo-electron microscopy.
As also discussed further below in the example section, example 2 shows that surprisingly IgE antibodies have a very rigid structure. Example 3 shows that such a rigid structure is also present when the IgE antibody is bound to an antigen (exemplified by GFP). Example 6 shows how structure determination using the method of the invention are performed for cryo-EM.

In a preferred embodiment, the method is for determining the structure of a target molecule. However, the structure of the interface between the antibody and the target molecule may also be determined. Thus, in another preferred embodiment, the method is for structure determination of an IgE antibody interacting with the target molecule. Such antibody/antigen interface structure determination may be relevant e.g. for therapeutics where improvement in antigen-antibody interactions may be based on the structure of the antigen-antibody interface. Thus, in yet an embodiment, the method is for structure determination of the binding site between an IgE antibody and the target molecule.

Different types of target molecules may have their structure determined by the method of the invention. In a preferred embodiment, the target molecule is a protein. In yet an embodiment the protein has at length in the range 10-5000 amino acids. Certain types of proteins may be particular relevant targets for structure determination. Thus, in yet an embodiment, the protein is selected from the group consisting of membrane spanning proteins, soluble proteins, and protein-nucleic acid complexes. In yet an embodiment the protein is selected from the group consisting of G-protein coupled receptors, single-pass transmembrane proteins, ion channels, active and passive transporters, receptors and pattern recognition receptors.

In yet an embodiment, the target molecule is a polynucleotide, such as RNA or DNA. In a related embodiment the polynucleotide has a length in the range of 20-2000 nucleotides. The target molecule may also be a chemical or natural compound. Thus, in yet an embodiment the target molecule has a molecular weight in the range 1000-10000 Da.

Standard EM structure determination methods (including cryo-EM) have limitations, when it comes to smaller target molecules. Thus, in an embodiment the target molecule for which the structure is to be determined has a molecular weight of less than 200 kDa, such as less than 150 kDa, such as less than 100 kDa, such as less than 50 kDa, such as less than 10 kDa or such as in the range 5-200 kDa, preferably in the range 5-50 kDa. Using the method of the invention the size limitation is avoided by including the weight of the IgE antibody or fragments or derivatives thereof. In addition, due to the identified rigid structure of IgE's, the complex formation between IgE and the target molecule does not interfere with particle averaging. Thus, in yet an embodiment, said complex of the target molecule and IgE antibody or fragment or derivative thereof, is at least 200 kDa, such as at least 250 kDa, such as at least 270 kDa, preferably such as at least 300 kDa.

Using e.g. cryo-EM it is possible to perform structure determination at high resolution allowing the construction of an atomic model. Thus, in an embodiment, the structure of the target molecule is determined at a maximum resolution higher than 5 Å such as higher than 4.5 Å, such as higher than 4 Å, such as higher than 3 Å or such as higher than 2 Å. In this context it is understood that "higher resolution" is equivalent to a lower numerical value with the unit Å.

The fragment or derivative of the IgE may comprise different structural elements to maintain the desired function. Thus, in an embodiment, said fragment or derivative of IgE comprises at least:
- the ε1 domain and the ε2 domain of IgE; or
- the ε1 domain, the ε2 domain, and the ε3 domain of IgE; or
- the ε1 domain, the ε2 domain the, ε3 domain and the ε4 domain of IgE;

It is of course important that the IgE comprises sufficient structural elements to maintain the desired function. Thus, in an embodiment said fragment or derivative of IgE is capable of forming a complex with the target molecule. Thus, in an embodiment, said fragment or derivative of IgE increases complex size, and/or increases rigidity of the complex and/or induces local two-fold symmetry of the complex and/or induces global two-fold symmetry of the complex.

It is well-known in the art to graft/replace antigen binding parts of an antibody with an alternative binding part (Antibody humanization methods - a review and update, Biotechnol Genet Eng Rev. 2013;29:175-86). The antigen binding part might derive from other antibody isotypes such as IgG, IgA, IgM, IgD and might derive from animal-derived antibodies of the IgE isotype or other antibody isotypes. The said antigen binding part comprises at least one complementarity determining region (CDR), or 2, 3, 4, 5, 6 CDRs (preferably 6 CDRs). The antigen binding part may comprise at least one variable region or, or both variable regions, or the entire light chain or the entire Fab. Thus, in an embodiment said fragment or derivative comprises a non-IgE binding domain having affinity for the target molecule.

Different types of IgE may find use in the method according to the invention. Thus, in an embodiment, the IgE antibody or fragment or derivative thereof, is selected from the group consisting of mammalian IgE, such as human IgE, mouse IgE, rat IgE, dog IgE, preferably human IgE. As shown in example 2, high rigidity is observed for two different recombinant IgE molecules, the HMM5-IgE and the H10-IgE, with distinct origin and antigen specificities expressed in either mammalian or insect cells. In example 3, high rigidity is shown for a complex of IgE and GFP. Overall this suggests that rigidity is a general feature of IgE molecules.

In yet an embodiment, the IgE antibody or fragment or derivative thereof is a monoclonal antibody.

The step of structure determination of the method may use different EM technologies. Thus, in an embodiment, said electron microscopy is selected from cryo-electron microscopy and negative stain electron microscopy. In examples 2-5 negative stain electron microscopy is used, whereas cryo-EM is used in example 6.

Different steps may be used dependent on the type of structure determination. In the case of cryo-electron microscopy specific steps may be applied. Thus, in an embodiment the step of determining the structure of the target molecule using cryo-electron microscopy, comprises one or more of the following steps, preferably all of the steps of
- applying an aqueous sample comprising the complex to a copper or gold holey carbon cryo-EM grid, wherein the sample is either free in the aqueous phase, suspended in the cryo-EM grid or adhered to a solid substrate applied to the cryo-EM grid, such as graphene, graphene oxide or a thin layer of amorphous carbon;
- freezing the sample while avoiding ice formation, e.g. by plunge freezing into liquid ethane;
- imaging the cryo-EM grid in an electron microscope under cryogenic temperatures, such as 10-173K, such as 50-150K, such as 70-110K, preferably in the range 90-100K, and
- structure-determination from the collected cryo-EM micrographs, e.g. when suitable, utilizing local or global symmetry to improve reliability and resolution of the resulting cryo-EM 3D reconstructions.

In the case of negative stain electron microscopy, specific steps may be applied. Thus, in an embodiment, the step of determining the structure of the target molecule using negative stain electron microscopy, comprises one or more of the following steps, preferably all of the steps of:
- applying an aqueous sample comprising the complex to an EM grid such as a copper grid with a layer of amorphous carbon;
- embedding the sample in a heavy metal stain, such as uranyl formate, uranyl acetate, tungstate methylamine or tungsten oxide;
- imaging the EM grid in an electron microscope;
- structure determination from the collected EM micrographs and when appropriate, utilizing local or global symmetry to improve reliability and resolution of the resulting negative stain EM envelopes.

The structure determination step may comprise only structure determination of the target molecule, however, often the structure of both the target molecule and the IgE (or at least part thereof) will be determined. Thus, in an embodiment, the structure of the target molecule and at least part of the IgE, or all of the IgE is determined.

The binding between the antibody and the target molecule (antigen) may take place by one or more interactions as well known for antibody-antigen interactions. Thus, in an embodiment, the binding between the IgE antibody or derivative or fragment thereof and the target molecule is through noncovalent interactions, such as electrostatic interactions, hydrogen bonds, Van der Waals interactions, and/or hydrophobic interactions. In yet an embodiment, the binding between the IgE antibody or derivative or fragment thereof and the target molecule involves the CDR regions of the antibody.

It could also be foreseen that a linker/adapter molecule could be positioned between the IgE and the target molecule to facilitate binding. Thus, in an embodiment, the binding between the IgE antibody or derivative or fragment thereof and the target molecule is facilitated or directly mediated through a linker/adapter molecule, preferably a non-covalently bound linker/adapter molecule. An advantage of the linker/adapter is that a single IgE antibody with specificity for the adapter can be used. Then only the adapter has to be partly modified to bind to different target molecules to be structurally determined. In an embodiment, the linker/adapter molecule is in contact with the IgE through the CDR regions of the IgE. As an example the GFP molecule in example 3 would act as a linker/adapter if the complex comprised another target molecule bound to the GFP. In a second embodiment, the IgE recognizes constant regions of the adapter molecule whereas variable regions of the adapter molecule contacts the target molecules in a rigid manner. In an embodiment, the adapter molecule is selected from the group consisting of antibody fragments, single domain antibodies, Nanobodies, aptamers based on oligonucleotides or other frameworks for molecular evolution, preferably Nanobodies. Example 7, elucidates how adapter molecules in the form of Nanobodies can be used in a method according to the invention.

The strength of interaction between the antibody and target molecule may also vary. Thus, in an embodiment, the binding affinity for the IgE antibody or fragment or derivative thereof to the target molecule is at the most 10 µM, such as at the most 5 µM, such as at the most 1 µM, under physiological conditions.

As also outlined above, the complexing of IgE to the target molecule has several advantages. Thus, in an embodiment, said step of complexing is for increasing complex size, and/or for increasing rigidity of the complex and/or for inducing local two-fold symmetry and/or inducing global two-fold symmetry of the complex.

As also outlined above, for structure determination using EM, global two-fold symmetry is a great advantage. Thus, in yet an embodiment, said complex further comprises a binding agent for an ε-domain of IgE, preferably for the ε2 and or the ε3 domain. As shown in example 4, when ligulizumab is bound to IgE apparent global two-fold symmetry is obtained rather than local two-fold symmetry as seen for IgE alone and the IgE-GFP complex (see examples 2 and 3). Thus, in yet an embodiment, the binding agent for an ε-domain of IgE is an antibody or fragment thereof, such as a Fab fragment, a F(ab')2, a Fv, a Fd, a dAb, a scFv fragment or a single-domain antibody (sdAb) such as a Nanobody, an aptamer, darpins, affibodies, chemical compounds or a lectin. In yet an embodiment, the binding agent for an ε-domain of IgE is selected from the group consisting of ligelizumab (ligelizumab fab), an anti-epsilon-Fab, and the 8D6 antibody (8D6 Fab). Phrased in another way, ligelizumab (fab) (and similar molecules as listed above) may be considered a global two-fold symmetry inducer of the complex. Example 5 shows global symmetry of a GFP-IgE-ligelizumab Fab complex.

In a further embodiment, the method comprises an additional step of binding a global two-fold inducing agent of the IgE to the Fc part of IgE. In yet a further embodiment said additional step may take place before, during or after binding of the target molecule to the IgE.

In an embodiment the molar ratio of the global two-fold inducing agent to IgE antibody or fragment or derivative thereof is in the range 10:1 to 1:10, such as 5:1 to 1:1 preferably 2:1. 2:1 will be the case where one IgE antibody comprises two epitopes for two global two-fold symmetry inducers (such as ligelizumab fab) (see example 4).

In yet an embodiment, the target molecules to have their structure determined are bound to the IgE antibody or fragment or derivative thereof at a ratio of target molecule to antibody or fragment or derivative thereof is in the range 10:1 and 0.1:1, such as 5:1 to 1:1 preferably 2:1. 2:1 will be the case where one IgE antibody binds two target molecules (such as GFP) (see example 3).

### Use of rigid structure of IgE's for structure determination of target molecules

As outlined above, it has surprisingly been found that IgE antibodies adopt a rigid structure, which may be a particular useful feature in structure determination of target molecules, when using electron microscopy. Thus, an aspect of the invention relates to the use of an IgE antibody or derivative or fragment thereof as a binding agent to a target molecule for which the structure is to be determined by electron microscopy. In an embodiment, said use is for increasing complex size, and/or for increasing rigidity of the complex and/or for inducing local two-fold symmetry of the complex or for inducing global two-fold symmetry of the complex.

In an embodiment, the IgE antibody or fragment or derivative thereof, is selected from the group consisting of mammalian IgE, such as human IgE, mouse IgE, rat IgE, dog IgE, preferably human IgE.

### Use of a binding agent for an ε-domain of IgE to induce global two-fold symmetry

As outlined above and also shown in examples 4 and 5, a binding agent for an ε-domain of IgE may induce global two-fold symmetry of the complex. Global two-fold symmetry of the complex is advantageous for structure determination when using EM. Thus, an aspect of the invention relates to the use of a binding agent for ε-domains of IgE, such as for the ε2 and/or the ε3 domain and/or ε4 domain, preferably for the ε2 and/or the ε3 domain for inducing global two-fold symmetry of a complex of the IgE and target molecule bound thereto.

In an embodiment, the binding agent (or global two-fold symmetry inducer), is selected from the group consisting of an antibody or fragments thereof, such as a Fab fragment, a F(ab')2, a Fv, a Fd, a dAb, a scFv fragment or a single-domain antibody (sdAb) such as a Nanobody, an aptamer, darpins, affibodies, chemical compounds and lectins.

In an embodiment, the binding agent for an ε-domain of IgE is selected from the group consisting of ligelizumab (fab), an anti-epsilon-Fab, and the 8D6 antibody (fab).

### Cryo-EM grid

As outlined above, the complexing of IgE or fragment or derivative thereof to a target molecule, may be important for structure determination of the target molecule using EM. Thus, an aspect of the invention relates to a cryo-EM grid loaded with a complex of IgE antibody or fragment or derivative thereof and a target molecule.

In an embodiment, the complex further comprises an agent (global two-fold symmetry inducer) bound to an ε-domain of IgE, preferably to the ε2 and/or the ε3 domain. In yet an embodiment, the agent (global two-fold symmetry inducer), is selected from the group consisting of an antibody or fragments thereof, such as a Fab fragment, a F(ab')2, a Fv, a Fd, a dAb, a scFv fragment or a single-domain antibody (sdAb) such as a Nanobody, an aptamer, darpins, affibodies, chemical compounds and lectins.

In an embodiment, the agent bound to the ε-domain of IgE is selected from the group consisting of ligelizumab (fab), an anti-epsilon-Fab, and the 8D6 antibody (fab).

In a further embodiment, the cryo-EM grid is loaded in a (cryo-) electron microscope.

It should be noted that embodiments and features described in the context of one of the aspects of the present invention also apply to the other aspects of the invention.

All patent and non-patent references cited in the present application, are hereby incorporated by reference in their entirety.

The invention will now be described in further details in the following non-limiting examples.

### Examples

### Example 1 - Materials and Methods

### Protein expression and purification

The HMM5 IgE antibody was selected locally and produced as IgE in insect cells and is specific for a well-known glycan epitope present on a variety of allergens, a modification of the core glycan by an 1,3-linked fucose. The H10 IgE (Christensen et al. J Allergy Clin Immunol. 2008 Aug;122(2):298-304) is cloned from a murine hybridoma line and reacts with the house dust mite allergen Der p 2. It exhibits high affinity for an epitope involving the N-terminus of the protein. The commercially available GFP-specific IgE (AbD18705_hIgE) is generated from a synthetic human repertoire. Both, the H10-IgE (locally produced) and the GFP-IgE (purchased from Bio-rad) were produced in human cell lines. The proteins were purified by affinity chromatography. The IgE complexes were reconstituted with the non-IgE component(s) in excess and purified by size exclusion chromatography.

### SAXS data collection and analysis

SAXS data collection of IgE was performed in batch mode at the BM29 beamline at the European Synchotron Radiation Facility (ESRF), Grenoble, France. The data were collected using a PILATUS 1M pixel detector and λ=0.992 Å in a temperature controlled capillary at 4°C. The sample-to-detector distance was 2.862 m, covering a range of momentum transfer q<4 nm⁻¹ (q=((4×π×sinθ)÷λ)) where 2θ is the scattering angle). Four SAXS measurements were performed on IgE in 20 mM HEPES, 50 mM NaCl, pH 7.2 between 0.3 and 1.5 mg/mL. The data were extrapolated to infinite dilution to remove any interparticle interference effects using ALMERGE. The molecular weight of IgE was estimated using BSA as a standard with I(0)=59.5 and a MW of 72 kDa, accounting for the monomer-dimer equilibrium. The pair distribution function was calculated by indirect Fourier Transform using GNOM.

### Negative stain EM sample preparation and data processing

Carbon evaporated copper grids (G400-C3) were glow-discharged for 45 seconds at 25 mA using an easiGlow (PELCO). 3 µL of IgE, IgE in complex with ligelizumab Fab or IgE-Fc in complex with ligelizumab Fab at 10-20 µg/mL was adsorbed to the grid for 5 seconds before being blotted away. The grid was washed twice in 3 µL of 20 mM HEPES pH 7.5, 50 mM NaCl followed by a staining step using a 3 µL drop of 2 % (w/v) uranyl formate allowing it to stain the grid for 45 seconds. The grids were imaged on a 120 kV Tecnai G2 spirit. Automated data collection was performed at a nominal magnification of 67.000x and a defocus ranging from -0.5 to -1.5 µm using leginon. Particles were picked using DoG picker in the Appion framework. 2D and 3D classification, initial model generation and 3D refinement was performed in RELION. Stochastic gradient descent (SGD) was used for initial model generation. For IgE in complex with ligelizumab, 3D classification and refinement were performed using C2 symmetry.

### Example 2 - Rigid IgE structure

### Aim of study

To show that IgE is rigid at the resolution of negative stain EM and adopts one major conformation.

### Materials and methods

See example 1.

### Results

Negative stain EM results showed that IgE is rigid and adopts one major conformation (Fig 2A-B). This rigid IgE structure is further supported by small angle x-ray scattering data (Fig 2C) which is important, as these measurements in contrast to nsEM are made under near-physiological conditions.

The observed IgE rigidity for two different recombinant IgE molecules (Fig 2A), the HMM5-IgE and the H10-IgE, with distinct origin and antigen specificities expressed in either mammalian or insect cells is important, as it suggests that rigidity is a general feature of IgE.

For comparison, an elaborate tomography study of IgG1 suggested 120 distinct conformations (Zhang X, et al. (2015) Sci Rep 5:9803). A few crystal structures of intact IgG antibodies where the conformation is stabilised by lattice contacts are known but also reveal a variety of overall conformations (Saphire EO, et al. (2002) J Mol Biol 319(1):9-18) and the conformations of IgG in this handful of crystal structures appear to be heavily influenced by crystal packing.

### Conclusion

In summary, it has been found that IgE's have a very rigid structure, which may be beneficial for EM structure determinations of target molecules. Isolated IgE possesses local two-fold symmetry that is beneficial during reconstruction of the 3D density.

### Example 3 - GFP binding to IgE

### Aim of study

To determine if the structure of GFP could be determined when complexed to IgE by increasing size and enabling local two-fold averaging.

### Materials and methods

See example 1.

### Results

Using EM, GFP binding to IgE was clearly observed. The antigen (GFP) bound at the VH-VL end of the Fab (Fig 3A-B) and the volume around the bound 30 kDa GFP fits well with the volume around the 50 kDa Fab binding the GFP antigen. If IgE-antigen complexes were flexible with a hinge at the junction between the Fab and the ε2 domains, the volume around the antigen would appear smaller since the antigen is located at the far end of the Fab. The bound antigen would therefore experience a larger movement as compared to the Cε1 and CL domains of the Fab closer to the ε2 domains, but this is apparently not the case.

Figure 3C illustrates how local two-fold symmetry may be used to improve the 3D density. The IgE-antigen complex is divided into 2x3 so called masks which are related by local two-fold symmetry each defined by a single rotation and a single translation operator. As the symmetry is only local, these three sets of operators are distinct from each other.

### Conclusion

This IgE-GFP complex data further show that the IgE rigidity is also a feature of IgE-antigen complexes and that structures of a target molecule bound to the IgE CDR regions can be determined be electron microscopy.

### Example 4 - IgE -ligelizumab Fab complex

### Aim of study

The discovery that IgE behaves well in negative stain EM lead to the investigation of the complex between IgE and ligelizumab. Ligelizumab is a second-generation anti-IgE antibody, which prohibits the formation of the IgE - FcεRI complex and thereby is intended to inhibit allergic responses in vivo. The aim was to determine the structural consequence of ligelizumab binding to IgE Fc.

### Materials and methods

The formed IgE-ligelizumab Fab complex was formed by SEC and analyzed by nsEM analysis. See also example 1.

### Results

Although the 3D reconstruction is slightly distorted due to preferred orientations, clear densities were observed for both the ligelizumab Fab and the IgE Fc and Fab moieties (Fig. 4A). The ligelizumab Fabs interact mainly with CHε3 but may also interact with CHε2. The two Fab's of IgE are pointing away from the Fc and does not seem to be contacted by the ligelizumab Fab. The extended conformation of IgE Fc observed in a complex between IgE and an anti-ε-chain antibody fits well with the negative stain EM envelope (Fig. 4A).

As demonstrated in figure 4A, implementation of global two-fold symmetry is applicable for this complex in contrast to free IgE and the IgE-GFP complex. Thus, an advantage of including ligelizumab in the complex is that apparent global two-fold symmetry is obtained rather than local two-fold symmetry (see examples 2 and 3).

### Conclusion

The Fab of IgE is pointing away from the Fc and does not seem to be obstructed by the ligelizumab Fab. The advantage of including ligelizumab in the complex is that an apparent global two-fold symmetry is obtained, rather than the local two-fold symmetry observed for IgE and IgE-GFP.

An advantage of global two-fold symmetry for EM and in for particular cryo-EM is that by continuously applying the two-fold symmetry during the iterative 3D reconstruction and averaging, the reliability of the underlying alignment of particles is increased. This also allows for better removal of improper particles corresponding to e.g. contaminants, partially denatured or aggregated protein. Hence, two-fold averaging of two identical subunits increases the resolution of the final cryo-EM 3D reconstruction

### Example 5 - Structure determination of antigen using IgE and a global symmetry inducer

### Aim of study

The discovery that the ligelizumab Fab induces global two-fold symmetry of IgE led to investigation of ligelizumab Fab in complex with anti-GFP IgE bound to its antigen GFP. The aim was to investigate whether the global two-fold symmetry would lead to an improved 3D reconstruction around GFP.

### Materials and methods

The formed GFP-IgE-Ligelizumab Fab complex was formed by SEC and analysed by nsEM analysis. See also example 1.

### Results

The 3D reconstruction of GFP-IgE-ligelizumab Fab complex is very similar to the reconstruction obtained for the IgE-ligelizumab Fab complex except for the presence of GFP. Importantly, the GFP-IgE-ligelizumab Fab complex also displays global two-fold symmetry (data not shown). The use of two-fold symmetry leads to improved resolution of the GFP, as compared to performing the reconstruction without two-fold symmetry imposed. The density corresponding to the (IgE Fab - GFP) moiety is very similar in size to the one observed for the (IgE Fab - GFP) moiety in the IgE-GFP complex itself, where only local twofold symmetry is present.

### Conclusion

Ligelizumab Fab is also able to induce global two-fold symmetry of IgE bound to an antigen. This allows for more accurate alignment during 3D classification and leads to improved reconstruction of the antigen. This shows that it is feasible to determine IgE-Antigen structures using ligelizumab Fab to induce global two-fold symmetry.

### Example 6 - Cryo-EM

### Aim of study

To produce a 3D reconstruction by cryo-EM that allows us to build an atomic model of the H10 IgE in complex with the 14 kDa Der p2 antigen.

### Materials and methods

The H10 IgE is cloned from a murine hybridoma line and reacts with the house dust mite allergen Der p2. It exhibits high affinity for an epitope at the N-terminus of the protein. Recombinant Der p2 is prepared in bacteria. The IgE-Der p2 complex is reconstituted with and purified by size exclusion chromatography.

For cryo-EM solution containing the complex is applied to glow-discharged holey carbon grids, which are flash frozen in liquid ethane using a Vitrobot Mark III. After identification of suitable grids data collection is performed on a 300 kV Titan Krios equipped with a direct electron detector. Data processing is performed on a high performance computing cluster. Local symmetry averaging is carried out after each iteration of 3D refinement. Partial crystal structures are docked into the cryo-EM density map followed by model building and real space refinement against the map. Variants of Der p2 predicted by the structure not to be recognized by the H10 IgE are constructed by site directed mutagenesis and the interaction of H10 IgE with wildtype and variant Der p2 is evaluated by surface plasmon resonance and in basophil degranulation experiments.

### Results

Large single particles are picked from the optimized cryo-EM grids. A 3D reconstruction with an overall resolution of 3 Å is obtained. Local symmetry averaging is shown to increase the resolution of the 3D reconstruction. An atomic model for the IgE and Der p2 is constructed and refined against the 3D reconstruction. A comparison of the affinity and activity of the H10 IgE for Der p2 and the variants confirmed the molecular interface observed in the atomic model constructed based on the cryo-EM reconstruction.

### Conclusion

The successful structure determination of IgE in complex with a 14 kDa target molecule demonstrates the principle that IgE-complexes facilitate structure determination by cryo-EM of small target molecules.

### Example 7 - Adapter molecules

### Aim of study

To determine the structure of a target protein bound to an IgE bound adaptor molecule.

### Materials and methods

In this hypothetical example small single domain antibodies called Nanobodies are used. As compared to conventional antibodies they are faster, cheaper and easier to produce and their antigen affinity is fully comparable to that of conventional antibodies. Like conventional antibodies they have a conserved framework and variable complementarity determining regions that recognize the antigen. Hence, first an IgE binding the nanobody constant region without interfering with antigen binding is developed, but this can then be used for all possible nanobodies. Second, for each target molecule a nanobody is developed binding the target with high affinity. Third, a complex is formed between the anti-nanobody IgE, the target molecule specific nanobody and the target molecule. The complex is isolated SEC and is analysed by negative stain EM or cryo-EM.

### Results

The structure of nanobody-bound target molecule is determined in complex with the anti-nanobody IgE. The local symmetry is used to improve the 3D reconstruction of the (target molecule) complex.

### Conclusion

The use of an adaptor molecule allows for determination of an unlimited number of target molecule structures using only a single IgE specific towards the conserved framework of the adaptor molecules. The increased size and local twofold symmetry allows for more reliable 3D reconstruction.

### Summary of data

In sum, the presented data shows that the IgE-(ligelizumab)₂ complex is a highly rigid module that allows structures of Ag₂-IgE-FabX₂ complexes to be determined by cryo-EM taking advantage of the size of the resulting complex (270 kDa+2×antigen Mw), the rigidity of IgE, and global two-fold symmetry. Thus, this concept transforms a standard reagent (antibody) into a vehicle for pipeline structure determination.

## Claims

1. A method for determining the structure of a target molecule and/or the structure of an IgE antibody interacting with the target molecule and/or the structure of the binding site between an IgE antibody and the target molecule, the method comprising
- providing a target molecule, for which the structure is to determined;
- providing an IgE antibody or fragment or derivative thereof, with binding affinity for the target molecule;
- binding the IgE antibody or fragment or derivative thereof to the target molecule, thereby producing a complex of the IgE antibody or fragment or derivative thereof and the target molecule; and
- determining the structure of at least the target molecule and/or the structure of an IgE antibody interacting with the target molecule and/or the structure of the binding site between the IgE antibody and the target molecule using electron microscopy, preferably cryo-electron microscopy.

2. The method according to claim 1, wherein the target molecule is a protein.

3. The method according to claim 1 or 2, wherein the target molecule, for which the structure is to be determined, has a molecular weight of less than 200 kDa, such as less than 150 kDa, such as less than 100 kDa, such as less than 50 kDa, such as less than 10 kDa or such as in the range 5-200 kDa, preferably in the range 5-50 kDa.

4. The method according to any of the preceding claims, wherein said complex of the target molecule and IgE antibody or fragment or derivative thereof, is at least 175 kDa, such as at least 200 kDa, such as at least 235 kDa, such as at least 270 kDa, preferably such as at least 300 kDa.

5. The method according to any of the preceding claims, wherein the structure is determined at a maximum resolution higher than 5 Å, such as higher than 4 Å, such as higher than 3 Å or such as higher than 2 Å.

6. The method according to any of the preceding claims, wherein said fragment or derivative of IgE comprises at least:
• the ε1 domain and the ε2 domain of IgE; or
• the ε1 domain, the ε2 domain, and the ε3 domain of IgE; or
• the ε1 domain, the ε2 domain the, ε3 domain and the ε4 domain of IgE;

7. The method according to claim any of the preceding claims, wherein said IgE antibody or said fragment or derivative of IgE increases complex size, and/or increases rigidity of the complex and/or induces local two-fold symmetry and/or induces global two-fold symmetry of the complex.

8. The method according to claim 6 or 7, wherein said fragment or derivative comprises a non-IgE binding domain having affinity for the target molecule.

9. The method according to any of the preceding claims, wherein said electron microscopy is cryo-electron microscopy.

10. The method according to any of the preceding claims, wherein the binding between the IgE antibody or derivative or fragment thereof and the target molecule is facilitated or directly mediated through a linker/adapter molecule, preferably a non-covalently bound linker/adapter molecule.

11. The method according to any of the preceding claims, wherein said step of complexing is for increasing complex size, and/or for increasing rigidity of the complex and/or for inducing local two-fold symmetry and/or inducing global two-fold symmetry of the complex.

12. The method according to any of the preceding claims, wherein said complex further comprises at least one binding agent, preferably two, for the heavy chain domains of IgE, preferably for the ε2 and/or the ε3 domains.

13. Use of an IgE antibody or derivative or fragment thereof as a binding agent to a target molecule, for which the structure is to be determined, by electron microscopy.

14. Use of a binding agent for one or more ε-domains of IgE, such as for the ε2 and/or the ε3 domain and/or ε4 domain, preferably for the ε2 and/or the ε3 domain for inducing global two-fold symmetry of a complex of the IgE and target molecule bound thereto.

15. A cryo-EM grid loaded with a complex of IgE antibody or fragment or derivative thereof and a target molecule.
